# FASCICULE DE BREVET EUROPEEN

(11) **EP 3 089 968 B1**
(45) Date de publication et mention de la délivrance du brevet: **07.02.2018**
(21) Numéro de dépôt: 14828171.0
(22) Date de dépôt: 30.12.2014
(51) Int. Cl.: C07D 317/26, C07D 493/08

(54) **PROCEDE POUR LA SYNTHESE INDUSTRIELLE DE LA SORDIDINE**
VERFAHREN ZUR INDUSTRIELLEN SYNTHESE VON SORDIDIN
PROCESS FOR THE INDUSTRIAL SYNTHESIS OF SORDIDIN

(30) Priorité: 30.12.2013 FR 1363699
(43) Date de publication de la demande: 09.11.2016
(73) Titulaire: Melchior Material and Life Science France, 64170 Lacq (FR)
(72) Inventeur: DUFOUR, Samuel, F-64300 Orthez (FR); LEJEUNE, Valérie, F-30230 Bouillargues (FR); SEVESTRE, Hubert, F-64150 Mourenx (FR)
(74) Mandataire: Regimbeau
(86) Numéro de dépôt international: PCT/EP2014/079470
(87) Numéro de publication internationale: WO 2015/101636

(56) Documents cités:
- DUCROT PAUL-HENRI: "Efficient synthesis of Sordidin, a male pheromone compound emitted by Cosmopolites Sordidus", SYNTHETIC COMMUNICATIONS., vol. 26, no. 21, 1996, pages 3923-3928, XP008172086, CHMARCEL DEKKER, INC., BASEL. ISSN: 0039-7911
- STEVE LANNERS ET AL.: "A convergent strategy for the Pamamycin Macrodiolides:", ANGEWANDTE CHEMIE. INTERNATIONAL EDITION., vol. 46, 2007, pages 7086-7089, XP002729746, DEVCH VERLAG, WEINHEIM. ISSN: 0570-0833
- HARUO YAMADA ET AL.: "New synrhetic intermediate for trans-8-methylhydrindanones", TETRAHEDRON LETTERS., vol. 31, no. 17, 1990, pages 2407-2410, XP002729747, NLELSEVIER SCIENCE PUBLISHERS, AMSTERDAM. ISSN: 0040-4039
- SEETHARAMAN JAYARAMAN ET AL.: "Synthesis, analysis, and field activity of Sordidin, a male-produced pheromone of the banana weevil", JOURNAL OF CHEMICAL ECOLOGY, vol. 23, no. 4, 1997, pages 1145-1161, XP002736081, SPRINGER, NL

## Description

La présente invention concerne un procédé d'obtention de la sordidine comprenant un procédé de préparation d'un intermédiaire réactionnel à des fins de synthèse industrielle de la sordidine, phéromone d'agrégation émise par le charançon noir mâle du bananier *(Cosmopolites sordidus).*

Le charançon « *Cosmopolites sordidus* » est l'insecte le plus dévastateur des plantations de bananes et il est répandu dans le monde entier. La sordidine de synthèse est utilisée dans des pièges à puits (associée à un insecticide ou à un pathogène) placés sur les sols des bananeraies, afin de réduire et contrôler les populations de charançons. Etant donné les volumes susceptibles d'être utilisés pour traiter les bananeraies, il est important de pouvoir synthétiser de manière industrielle à partir de matières premières abondantes et/ ou peu chères et à moindre coût ce composé phéromone. La présente invention a pour objet un nouveau procédé de synthèse industrielle d'un intermédiaire clé de la sordidine.

La sordidine de formule chimique 2,8-dioxa 1-éthyl 3,5,7- triméthyl bicyclo(3,2,1) octane possède dans sa structure chimique quatre centres chiraux en positions 1,3,5 et 7. Du fait de sa structure bicyclique (cf. schéma1), il existe quatre isomères a, b, c et d qui se différencient par la configuration absolue des carbones en positions 3 et 7, la configuration des centres 1 et 5 étant figée.

L'isomère d (1S, 3R, 5R, 7S) correspond à la phéromone naturelle de *« Cosmopolites sordidus »,* il a été isolé et sa structure caractérisée pour la première fois en 1995 (J. Beauhaire, Tetrahedron Letters, 36 (7) 1043-1046, 1995) (cf. schéma 1)

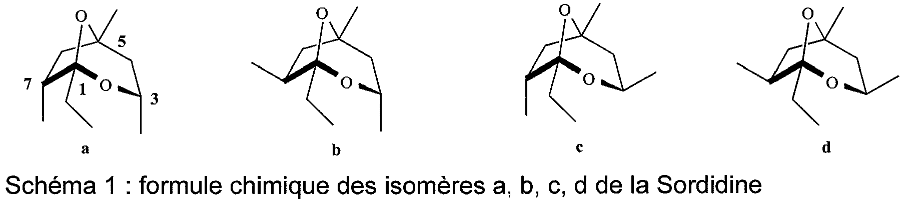

L'asymétrie des molécules est importante tant du point de vue chimique que biologique (K. Mori, Biosci . Biotech. Biochem., 60 (12) 1925-1932, 1996). Par exemple, pour certains insectes, un seul stéréo-isomère peut être bioactif et les autres peuvent inhiber l'action de la phéromone. Dans les effluves émises par le charançon du bananier, on ne trouve de la sordidine que sous la forme du seul stéréo-isomère d (1S, 3R, 5R, 7S) (K. Mori, Tetrahedron Lett., 37 3741-3744, 1996), cependant les études sur le terrain ont montré que le mélange racémique des 4 stéréo-isomères reste bioactif (I. O. Ndiege, Naturwissenschaften, 83 280-282 1996). En conséquence, dans le cas de la sordidine, les procédés de synthèse conduisant à des énantiomères purs (D. J. Wardrop, Tetrahedron Lett., 43 737-739, 2002 ; D. Enders, Eur. J. Org. Chem, 2677-2683 2005, et J.S. Yadav, Tetrahedron, 64 2063-2070, 2008) ne sont pas les plus intéressants car leur mise en oeuvre est complexe et fait intervenir des catalyseurs onéreux, pour un gain applicatif limité.

Un procédé de synthèse racémique a été décrit par Ducrot (P.H. Ducrot, Synth. Comm., 26 (21) 3923-3928, 1996). Cette synthèse a pour point de départ une matière première peu coûteuse : la 3-pentanone (notée 1 sur le schéma 2), contrairement à la synthèse proposée par Jayaraman (S. Jayaraman, J. Chem. Ecol., 23 1145-1161, 1997) qui utilise une matière première peu accessible industriellement.

Le procédé de synthèse selon Ducrot comporte douze étapes. La demanderesse entend par le mot étape toute réaction chimique et son traitement conduisant à l'isolement d'un intermédiaire.

Au cours du procédé de Ducrot, à partir de la 3-pentanone et après 2 étapes réactionnelles, est synthétisé l'intermédiaire oléfine suivant : 2-éthyl-2-(pent-4-èn-2-yl)-1,3-dioxolane (composé 3 ci-dessous).

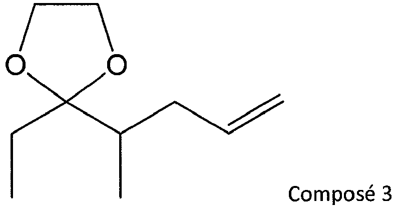

Selon le procédé décrit par Ducrot, ce composé 3 est ensuite transformé via 6 autres étapes en la cétone correspondante (composé 4 ci-dessous) : 4-(2-éthyl-1,3-dioxolan-2-yl)pentan-2-one.

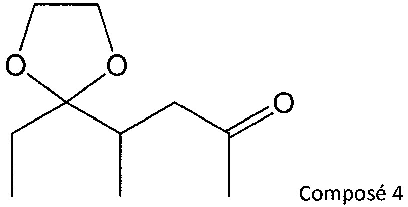

Le composé 4 est à son tour transformé en sordidine à la suite de 4 étapes réactionnelles telles que décrites par Ducrot.

Il est à noter que les six étapes de transformation du composé 3 en 4 entraînent non seulement une chute du rendement global de la synthèse mais elles utilisent aussi des réactifs non recommandés à l'échelle industrielle dont notamment le tétroxyde d'osmium (OSO₄) très toxique.

Lanners et al (Angewandte Chemie, international édition., vol 46, 2007, pages 7086-7089, DEVCH VERLAG Weinheim) et Yamada et al (Tetrahedron Letters, vol 31, N°17, 1990, pages 2047-2410) décrivent des réactions d'oxydation de dérivés pent-4-ène-2-yl-3-dioxolane différents pour la préparation de dérivés (4-dioxolan-2-yl)-pentan-2-one correspondants en présence de catalyseur comprenant un complexe organometallique de métaux de transition, d'oxygène et d'eau comme solvant. Cependant aucun des deux n'a rapport avec la préparation de sordidine.

Il apparaît ainsi qu'il existe un besoin en un procédé de synthèse de la sordidine qui n'implique qu'un nombre le plus réduit possible d'étapes réactionnelles permettant l'obtention de sordidine avec des rendements industriellement optimaux et tout en évitant autant que faire se peut l'utilisation de réactifs et/ou solvants onéreux, dangereux ou hautement nocifs.

La présente invention se propose donc de fournir un nouveau procédé de synthèse de la sordidine impliquant un nombre réduit d'étapes réactionnelles, ledit procédé comprenant une nouvelle voie de synthèse d'un intermédiaire.

A cet effet, la présente invention concerne ainsi un procédé de préparation de la sordidine comprenant une étape de préparation de la 4-(2-éthyl-1,3-dioxolan-2-yl)pentan-2-one (composé 4) 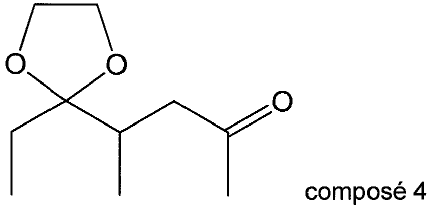 par réaction d'oxydation de la 2-éthyl-2-(pent-4-èn-2-yl)-1,3-dioxolane (composé 3) 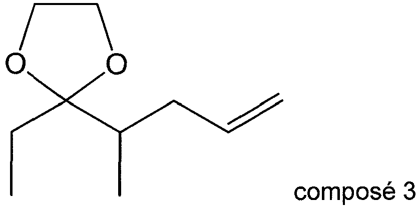 en présence d'un catalyseur choisi dans le groupe comprenant les complexes organométalliques de métaux de transition.

Dans un mode particulier de réalisation, le procédé selon la présente invention comprend la transformation du composé 4 en composé 5 par couplage magnésien avec l'halogénure d'allyle magnésium, puis de la transformation du composé 5 en composé 6 par oxydation, par exemple avec un peracide tel que l'acide péracétique, l'acide perpropionique ou l'acide métachloroperbenzoïque, puis transformation du composé 6 en composé 7 par ouverture d'époxyde, par exemple avec un hydrure métallique, et conversion du composé 7 en sordidine par cyclisation, par exemple en milieu acide.

La présente invention permet ainsi d'obtenir la sordidine via un procédé dans lequel le composé 4 est obtenu en une seule étape à partir du composé 3.

Le présent procédé permet ainsi de réduire considérablement le nombre d'étapes lors de la synthèse de la sordidine par rapport à un procédé tel que décrit par Ducrot.

En effet, dans le procédé divulgué par Ducrot, la conversion du composé 3 en composé 4 implique pas moins de six étapes alors que le procédé selon la présente invention permet le passage du composé 3 au composé 4 en une seule étape. Ainsi le rendement global de synthèse de la sordidine selon l'invention, comprenant le procédé d'obtention du composé 4 par réaction d'oxydation du composé 3 en présence d'un catalyseur choisi dans le groupe comprenant les complexes organométalliques de métaux de transition, est considérablement augmenté.

De plus, les solvants mis en oeuvre, seuls ou en mélange, dans l'étape de conversion du composé 3 en 4, tels que l'eau, l'acétone ou le DMF par exemple, sont considérablement moins dangereux et mieux adaptés à des développements industriels que les solvants décrits par Ducrot (THF et CH₂Cl₂).

Le procédé selon la présente invention présente donc l'avantage précieux d'améliorer le rendement de la réaction globale de synthèse de la sordidine à partir du composé 3 tout en mettant en oeuvre des solvants peu ou pas nocifs tans vis-à-vis des opérateurs que de l'environnement. Enfin, un tel procédé selon l'invention est aisément industrialisable.

Le schéma 2 ci-dessous résume le procédé d'obtention de la sordidine comprenant le procédé d'obtention du composé 4 à partir du composé 3, selon la présente invention.

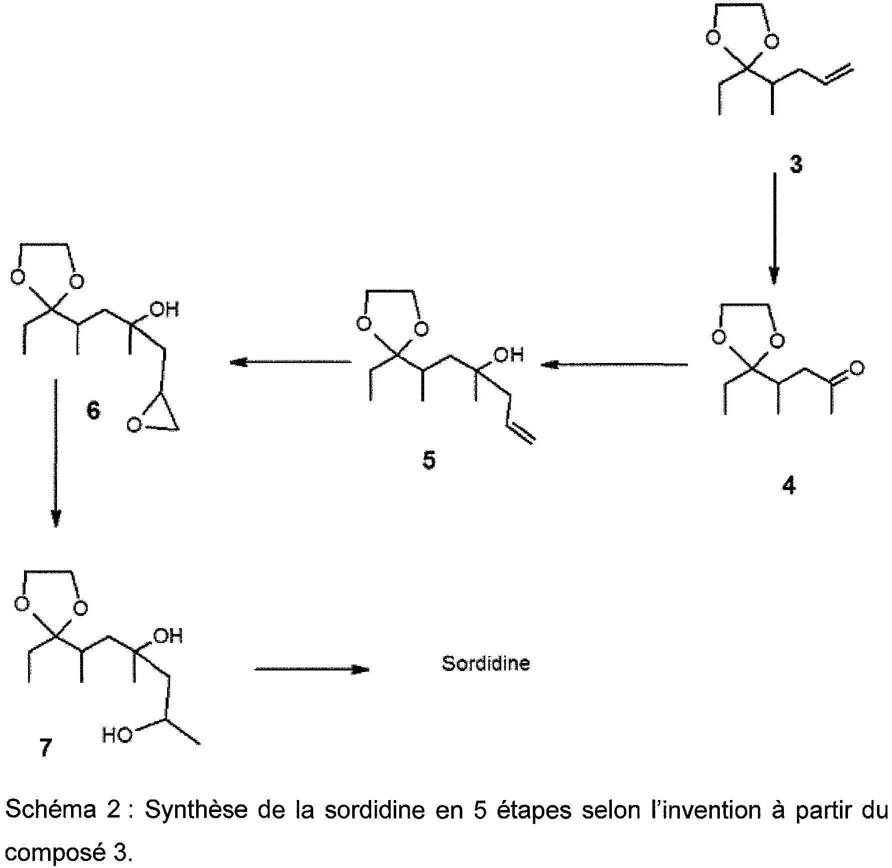

L'objet de l'invention est donc un procédé de synthèse de la sordidine comprenant une étape permettant d'obtenir le composé 4 à partir du composé 3 via une réaction d'oxydation en présence d'un catalyseur choisi parmi les complexes organométalliques de métaux de transition

Dans un mode de réalisation de l'invention, la réaction d'oxydation du composé 3 en composé 4 est menée en présence d'un agent oxydant choisi parmi l'air, l'air enrichi en oxygène, l'oxygène O₂ et les hydropéroxydes de formule générale R-OOH dans laquelle R peut être un atome d'hydrogène, un groupement alkyle en C₁-C₆ linéaire ou ramifié, particulièrement un groupement terbutyl ou un groupement aromatique cumyle.

Dans ce mode de réalisation particulier de l'invention, l'agent oxydant de la réaction d'oxydation du composé 3 en composé 4 pourra être choisi parmi l'air, l'oxygène, le tBuOOH ou le H₂O₂.

Dans un mode de réalisation de l'invention le catalyseur de la réaction d'oxydation du composé 3 en composé 4 est choisi parmi les complexes organométalliques de métaux de transition. Dans un tel mode de réalisation particulier de l'invention, le catalyseur de la réaction d'oxydation du composé 3 en composé 4 pourra être complexé par un ligand choisi parmi les amines, phosphines et phtalocyanines.

Dans un mode particulier de réalisation de l'invention, le catalyseur de la réaction d'oxydation du composé 3 en composé 4 est choisi parmi les complexes organométalliques de métaux de transition à base de Ni, Rh, Ir, Pd, Co ou Pt.

Dans un mode particulier de réalisation le catalyseur de la réaction d'oxydation du composé 3 en composé 4 pourra être choisi parmi les composés du Palladium (II) tels que PdCl₂, Pd(Acétate)₂, Pd(Acétate)(triflate), Pd(OH)₂ ou PdBr₂. Dans un mode encore préféré, le catalyseur de la réaction d'oxydation du composé 3 en composé 4 est le Pd(Acetate)₂.

Dans un autre mode particulier de réalisation de l'invention, le catalyseur de la réaction d'oxydation du composé 3 en composé 4 pourra être choisi parmi les composés du Nickel (II) tels que Ni(Acétate)₂, NiCl₂ ou NiBr₂.

Dans un mode de réalisation de l'invention, le catalyseur de la réaction d"oxydation du composé 3 en composé 4 pourra être choisi parmi les composés du Cobalt Co(No₃)₂, CoCl₂, CoBr₂ ou Co(Acétate)₂.

Enfin, dans un autre mode de réalisation de l'invention, le catalyseur de la réaction d"oxydation du composé 3 en composé 4 pourra être choisi parmi les composés du Rhodium (III) tels que Rh(Cl) ou Rh(ClO₄)₃.

Dans un mode de réalisation de l'invention, le procédé selon l'invention comprend l'ajout d'un régénérateur du catalyseur de la réaction d"oxydation du composé 3 en composé 4, qui peut être choisi parmi les régénérateurs à base de cuivre ou de fer tels que CuCl, CuCl2, CuBr, CuBr2, CuAcétate, FeCl2, FeCl3.

Dans un tel mode de réalisation de l'invention, le régénérateur du catalyseur pourra être associé à un ou plusieurs ligands choisis parmi les phtalocyanines.

Dans un mode de réalisation particulier de l'invention, la réaction d'oxydation du composé 3 en 4 est réalisée en présence d'un solvant ou d'un mélange de solvants choisis parmi l'eau, les solvants alcooliques, les solvants acides, les solvants de type cétones, les solvants éthérés, les solvants azotés, ainsi que les solvants de type polymère liquide ou leur mélange.

Dans un mode particulier de réalisation de l'invention, le solvant alcoolique pourra être choisi parmi le méthanol (MeOH) ou l'alcool terbutylique (tert-BuOH).

Dans un mode de réalisation plus particulier, le solvant acide pourra être choisi parmi les solutions aqueuses d'acide chlorhydrique (HCl) ou d'acide perchlorique (HClO₄).

Dans un autre mode de réalisation, le solvant de type cétone pourra être choisi parmi l'acétone ou la méthyl éthyl cétone.

Dans un autre mode de réalisation, le solvant de type azoté pourra être choisi parmi la DMA (diméthylacétamide), la diméthyl formamide (DMF) ou la NMP (N-méthyl-2pyrrolidone).

Dans un autre mode de réalisation, le solvant de type éthéré pourra être choisi parmi le DME (diméthoxyéthane), le diglyme, ou le MTBE (méthyltertiobutyléther).

Dans un autre mode de réalisation, le solvant de type polymère liquide pourra être choisi parmi les polyéthylènes glycol de masse moléculaire moyenne en nombre inférieure à 1000 ou leur mélange.

Dans un mode de réalisation particulier de l'invention la réaction d'oxydation du composé 3 en composé 4 est réalisée en présence d'au moins un solvant choisi parmi l'eau, le DMF, le DMA, l'acétone, l'heptane, le tert-BuOH ou leur mélange

Le procédé selon la présente invention débute par l'obtention du composé 3 selon la méthode décrite par Ducrot à partir de réactifs chimiques aisément disponibles dans le commerce.

Le composé 3 est ainsi introduit, préférentiellement sous agitation, dans un réacteur adéquat et sont ajoutés le solvant ou le mélange de solvant, le catalyseur et l'éventuel régénérateur du catalyseur, l'agent oxydant ainsi qu'éventuellement le ligand du catalyseur ou du régénérateur.

Le milieu réactionnel est ensuite porté à une température qui peut être comprise entre la température ambiante et une température d'environ 100°C, ceci suivant la nature du solvant mis en oeuvre.

Dans un mode de réalisation de l'invention, l'oxydation du composé 3 en 4 pourra être réalisée à une température comprise entre environ 20°C et environ 100°C, particulièrement entre environ 25°C et environ 80°C, plus particulièrement entre environ 40°C et environ 80°C, plus particulièrement encore entre environ 50°C et environ 60°C.

Le milieu réactionnel peut être brassé et agité et la pression à l'intérieur du réacteur peut être augmentée de manière à mener la réaction à une pression comprise entre la pression atmosphérique et une pression d'environ 30 bars. Dans un mode de réalisation de l'invention, l'oxydation du composé 3 en 4 pourra être réalisée à une pression comprise entre la pression atmosphérique et environ 30 bars plus particulièrement entre environ 1 bar et environ 20 bars, plus particulièrement encore entre environ 1 bar et environ 10 bars, plus particulièrement encore aux environs de 5 bars.

Après un certain temps de réaction qui pourra être compris entre 1 et 20 heures, la réaction est stoppée par abaissement de la température et/ou de la pression au sein de l'enceinte réactionnelle.

Typiquement, la formation du composé 4 est suivie par Chromatographie en Phase Gazeuse afin d'évaluer l'avancement de la réaction. Le composé 4 peut être récupéré par extraction liquide-liquide et/ou décantation suivie d'une évaporation et d'une éventuelle distillation afin d'obtenir le composé 4 avec un degré de pureté adéquat pour poursuivre la synthèse et l'obtention de la sordidine.

Les ratios molaires de catalyseur par rapport au composé 3 pourront être compris entre 0,1% et 10%, de préférence entre 0,1%, et 5% et plus préférentiellement encore entre 0,1% et 1%. La quantité de régénérateur sera comprise entre 1 à 10 fois celle du catalyseur et celle d'un éventuel ligand sera d'1 équivalent molaire par rapport au catalyseur.

Ainsi la présente invention vise un procédé de préparation de la sordidine comprenant une étape de préparation du composé 4 par réaction d'oxydation du composé 3 tel que décrit précédemment suivi de la transformation du composé 4 en composé 5 par couplage magnésien avec l'halogénure d'allyle magnésium, puis de la transformation du composé 5 en composé 6 par oxydation, par exemple avec un peracide tel que l'acide péracétique, l'acide perpropionique ou l'acide métachloroperbenzoïque, puis transformation du composé 6 en composé 7 par ouverture d'époxyde, par exemple avec un hydrure métallique, et conversion du composé 7 en sordidine par cyclisation, particulièrement par cyclisation en milieu acide. Les différentes étapes permettant d'obtenir la sordidine à partir du composé 4 sont décrites en détail par Ducrot dans la publication suivante : P.H. Ducrot, Synth. Comm., 26 (21) 3923-3928, 1996.

La présente invention concerne aussi un procédé de préparation de la 4-(2-éthyl-1,3-dioxolan-2-yl)pentan-2-one (composé 4) 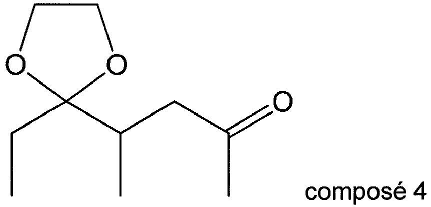 par réaction d'oxydation de la 2-éthyl-2-(pent-4-èn-2-yl)-1,3-dioxolane (composé 3) 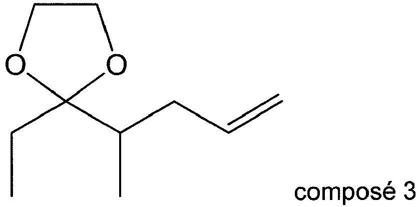 en présence d'un catalyseur choisi dans le groupe comprenant les complexes organométalliques de métaux de transition.

Ainsi, à partir de la 3-pentanone, en suivant le procédé selon la présente invention, la sordidine peut être obtenue en 7 étapes en lieu et place des 12 étapes enseignées par Ducrot. La réduction du nombre d'étapes et d'intermédiaires réactionnels selon le présent procédé impacte positivement le rendement global de la réaction de synthèse de la sordidine et permet en outre de s'affranchir de l'utilisation de réactifs dangereux, onéreux et/ou toxiques tel que le tétroxyde d'osmium.

Enfin un autre objet de l'invention consiste en une composition de sordidine présentant une distribution particulière des isomères de la sordidine grâce au procédé de préparation selon l'invention.

Ainsi la présente invention a pour objet une composition de sordidine caractérisée en ce que qu'elle comprend les 4 diastéréoisomères a, b, c et d (tels qu'indiqués au schéma 1) dont les proportions respectives mesurées par chromatographie en phase gazeuse sont comprises dans les intervalles : 31-36% ; 16-20% ; 16-20% ; 28-32%.

En particulier, une composition selon l'invention est une composition de sordidine caractérisée en ce que les proportions respectives des quatre diastéréoisomères a, b, c et d, mesurées par chromatographie en phase gazeuse, sont d'environ 34% ; 19% ; 16,9% et 30,1%.

Une telle composition peut être obtenue par un procédé selon la présente invention tel que décrit dans la présente description et qui permet d'obtenir en quantité majoritaire les isomères a et c.

La mesure des proportions des 4 diastéréoisomères a, b, c et d est réalisée par la mesure de la quantité de ces isomères via la mesure de l'aire sous courbe suite à une mesure par chromatographie en phase gazeuse dans des conditions telles que décrites dans la section Exemples, ci-après. Les pourcentages respectifs des 4 diastéréoisomères sont exprimés en rapport à la quantité totale de sordidine, tous isomères confondus.

### EXEMPLES

Le produit de départ, le composé 3 : 2-éthyl-2-(pent-4-en-2-yl)-1,3-dioxolane peut être obtenu selon la procédure de Ducrot, à partir de la 3-pentanone après deux première étapes décrites dans : P.H. Ducrot, Synth. Comm., 26 (21) 3923-3928, 1996.

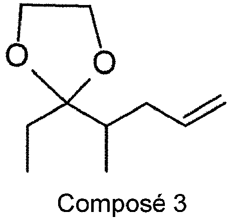

Les autres réactifs sont achetés auprès des fournisseurs classiques de la chimie.

### Analyse en Chromatographie en phase gazeuse

Le suivi réactionnel des différentes étapes de synthèse de la sordidine est effectué par Chromatographie en Phase Gazeuse (CPG). Les résultats sont exprimés en pourcentage relatif. Les quantités relatives de composés sont évaluées via la mesure de l'aire sous courbe. Les analyses ont été effectuées dans les conditions suivantes : appareil de CPG de marque Hewlett Packard (5890 Série II), équipé d'un détecteur FID (Flame Ionisation Detector) et d'une colonne HP5 (Agilent J&W) 30 m x 0,52mm Film 0,88 µm, avec une pression d'hélium de 3 à10 psi, une température d'injecteur de 250°C, une température de détecteur de 280°C. Ont aussi été utilisés : une température de four initiale de 50°C, un temps initial de 3 min une rampe 1 de 10°C/min, une température finale 1 de 260°C et un temps de palier 1 de 5 min.

Les échantillons à analyser ont été préparés en diluant 100 mg d'intermédiaire de synthèse ou de produit à analyser dans de l'acétonitrile (QSP 20 ml). Le volume d'échantillon injecté dans la CPG est de 1 µl.

### EXEMPLE 1

Réaction d'oxydation sous pression d'air dans les conditions : pression d'air (6 bars), catalyseur PdCl2 plus CuCI dans un mélange eau-DMF

Dans un ballon tricol de 250 ml équipé d'un palier d'agitation et d'un agitateur bipales sont introduits successivement 93,5 ml de DMF, 19,5 ml d'eau, 43 mg de chlorure de palladium, 295 mg de chlorure cuivreux et enfin 8.5 g de composé 3.

Le milieu réactionnel est chauffé à 80°C et de l'air comprimé est injecté entrainant une augmentation de pression du réacteur à 5 bars avec un léger renouvellement d'air. Après 8h30 d'agitation, 13% de conversion est observé par CPG et la pureté relative du composé 4 formé est de 11%. Le faux de conversion obtenu a amené les inventeurs à optimiser les conditions de réactions qui sont détaillées dans les exemples suivants.

### EXEMPLE 2

Réaction d'oxydation sous pression d'air dans les conditions : pression d'air (6 bars), catalyseur PdCl2 plus CuCl dans un mélange eau-DMA

Dans un réacteur de 70L, sont chargés sous agitation, successivement 44kg diméthylacétamide (DMA), 2,6kg du composé 3, 6,6 kg d'eau, 13,8g de chlorure de palladium, et 93g de chlorure cuivreux. Le milieu réactionnel est chauffé à 80°C et de l'air comprimé est injecté entrainant une augmentation de pression du réacteur à 6 bars avec un léger renouvellement d'air. La formation du composé 4 est suivie en chromatographie phase gazeuse dans les conditions décrites précédemment. Après 12h d'agitation, on constate que tout le produit de départ a disparu. Le milieu réactionnel est refroidi à température ambiante puis de l'eau est ajoutée (26kg). On observe une exothermie. Le composé 4 est ensuite récupéré par décantation suivie de quatre extractions au méthyl-tert-butyl éther MTBE (5V) et terminée par deux lavages à l'eau des phases organiques (1V). Après évaporation, les fractions sont rassemblées et distillées jusqu'à 72°C à 4mbar. On effectue une redistillation des fractions, jusqu'à l'obtention d'un liquide de couleur jaune pâle (2,2kg de composé 4 ,rendement 80%, pureté CPG=94%).

### EXEMPLE 3

Réaction d'oxydation sous pression atmosphérique d'air, dans les conditions : 60°C, catalyseur acétate de palladium dans l'acétone, oxydant t-butylhydropéroxyde

On introduit dans un ballon tricol de 250ml équipé d'un palier d'agitation et d'un agitateur bipales 100 ml d'acétone, 65,6ml de t-butylhydropéroxyde (tBuOOH) puis sous agitation 1,76g de catalyseur: l'acétate de palladium et enfin 27,93g de composé 3.

Le milieu réactionnel est chauffé à 60°C sous pression atmosphérique. La formation du composé 4 est suivie par chromatographie gazeuse.

Après 14h d'agitation (le taux de conversion est de 97,9%), le milieu réactionnel est refroidi à température ambiante.

On ajoute ensuite 100 ml d'une solution contenant 50g de de thiosulfate de sodium et 100 ml de MTBE. Le milieu réactionnel est filtré et après décantation la phase organique supérieure est récupérée. Après évaporation des solvants à 60°C, suivie d'une distillation sous vide à une pression de 2mbar, le composé 4, 11.1 g (36,3% de rendement) est obtenu sous la forme d'un liquide limpide jaune pâle avec une pureté relative de 90,7% par CPG.

### EXEMPLES 4 à 8

Les exemples 4 à 8 permettent d'illustrer les différentes conditions opératoires envisagées concernant l'étape de transformation du composé 3 en composé 4 dans le cadre de la présente invention.

Les différents procédés testés sont répartis en deux grands types, les essais sous pression qui suivent le protocole expérimental décrit dans l'exemple 2, les essais à pression atmosphérique qui suivent le protocole décrit dans l'exemple 3.

| | **Ex 1** | **Ex 2** | **Ex 3** | **Ex 4** | **Ex 5** | **Ex 6** | **Ex 7** | **Ex 8** |
|---|---|---|---|---|---|---|---|---|
| **Conditions type** | Ex 2 | Ex 2 | **Ex** 3 | Ex 2 | Ex 3 | Ex 3 | Ex 3 | **Ex** 3 |
| **Oxydant** | air | air | tBuOOH | Air enrichi | tBuOOH | H₂O₂ | tBuOOH | tBuOOH |
| **Catalyseur (et éventuel régénérateur)** | PdCl₂+ Cucl | PdCl₂+ Cucl | PdO (Acétate)₂ | PdCl₂+ Cucl | Pd (Acétate) (Triflate) | PdOAC₂ | PdOAC₂ | Pd(OAc)₂ + ligand NaOAC |
| **Solvant** | DMF+ eau | DMA +eau | Acétone | Acétone | Acétone | Tert-BuOH | Acétone | Heptane |
| **Température (°C)** | 80 | 80 | 60 | 80 | 50 | 50 | 60 | 98 |
| **Pression (atm)** | 5 | 6 | 1 | 5 | 1 | 1 | 1 | 1 |
| **Taux de conversion du composé 3 en 4** | 13% | 91% | 97% | 91% | 67% | 80% | 98% | >95% |

### EXEMPLE 9

A partir du composé 4, on obtient le composé 5 par réaction avec le chlorure d'allyl magnésium, puis le composé 6 par oxydation ménagée et ce dernier composé est ensuite obtenu par hydrolyse acide. La sordidine obtenue est caractérisée par chromatographie en phase gazeuse. On mesure la distribution des 4 diastéréoisomères a, b, c et d de la sordidine tels qu'indiqués au schéma 1. Les temps d'élution correspondent aux quatre diastéréoisomères tel que cela avait été démontré par Beauhaire (Tetrahedron Letters, 36 (7) 1043-1046, 1995).

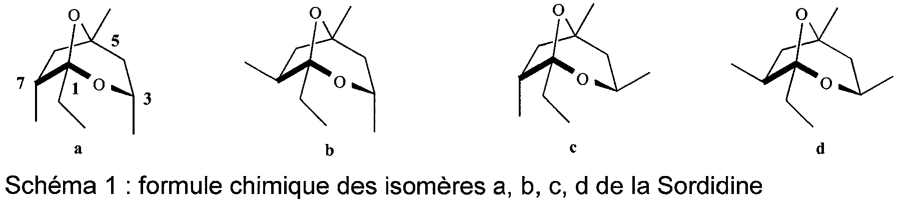

L'analyse de la quantité, par calcul de l'aire sous courbe, des 4 diastéréoisomères de sordidine contenus dans le mélange de sordidine obtenu donne la répartition suivante : a : 34% ; b : 19% ; c : 16,9% ; d : 30,1%.

## Revendications

1. Procédé de préparation de la sordidine comprenant une étape de préparation de la 4-(2-éthyl-1,3-dioxolan-2-yl)pentan-2-one (composé 4) 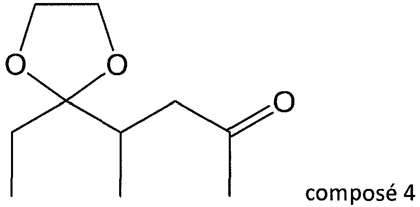 par réaction d'oxydation de la 2-éthyl-2-(pent-4-èn-2-yl)-1,3-dioxolane (composé 3) 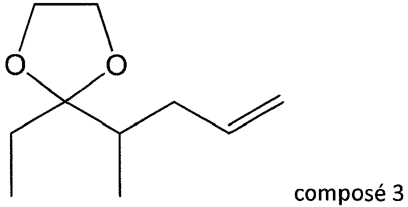 en présence d'un catalyseur choisi dans le groupe comprenant les complexes organométalliques de métaux de transition.

2. Procédé selon la revendication 1, **caractérisé en ce que** la réaction d'oxydation est menée en présence d'un agent oxydant choisi parmi l'air, l'air enrichi en oxygène, l'oxygène O₂ et les hydropéroxydes de formule générale R-OOH dans laquelle R peut être un atome d'hydrogène, un groupement alkyle en C₁-C₆ linéaire ou ramifié.

3. Procédé selon les revendications 1 ou 2, **caractérisé en ce que** le catalyseur est choisi parmi les complexes organométalliques de métaux de transition à base de Ni, Rh, Ir, Pd, Co ou Pt.

4. Procédé selon l'une des revendications précédentes, **caractérisé en ce que** le catalyseur est choisi parmi les composés du Palladium (II), parmi les composés du Nickel (II), parmi les composés du Cobalt ou encore parmi les composés du Rhodium (III).

5. Procédé selon l'une des revendications précédentes **caractérisé en ce que** le catalyseur est complexé par un ligand choisi parmi les amines, phosphines et phtalocyanines.

6. Procédé selon l'une des revendications précédentes, **caractérisé en ce que** l'on ajoute un régénérateur du catalyseur.

7. Procédé selon la revendication 6, **caractérisé en ce que** le régénérateur de catalyseur est choisi parmi les régénérateurs à base de cuivre ou de fer.

8. Procédé selon l'une des revendications 1 à 4 **caractérisé en ce que** le catalyseur est Pd(Acétate)₂.

9. Procédé selon l'une des revendications précédentes, **caractérisé en ce que** la réaction d'oxydation du composé 3 en composé 4 est réalisée en présence d'au moins un solvant choisi parmi l'eau, les solvants alcooliques, les solvants acides, les solvants de type cétones, les solvants éthérés, les solvants azotés, ainsi que les solvants de types polymères liquides.

10. Procédé selon l'une des revendications précédentes, **caractérisé en ce que** la réaction d'oxydation du composé 3 en composé 4 est réalisée en présence d'au moins un solvant choisi parmi l'eau, le DMF, le DMA, l'acétone, l'heptane, le tertBuOH ou leur mélange.

11. Procédé selon l'une des revendications précédentes, **caractérisé en ce que** la réaction d'oxydation est réalisée sous une pression comprise entre la pression atmosphérique et 30 bars.

12. Procédé selon l'une des revendications précédentes, **caractérisé en ce que** la réaction d'oxydation est réalisée à une température comprise entre 25°C et 100°C.

13. Procédé de synthèse de la sordidine **caractérisé en ce qu'**il comprend un procédé selon l'une des revendications précédentes, suivi de la transformation de la 4-(2-éthyl-1,3-dioxolan-2-yl)pentan-2-one (composé 4) 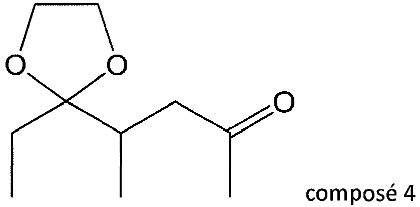 en composé 5 par couplage magnésien avec l'halogénure d'allyle magnésium, puis de la transformation du composé 5 en composé 6 par oxydation, puis transformation du composé 6 en composé 7 par ouverture d'époxyde, et conversion du composé 7 en sordidine par cyclisation.

14. Procédé de préparation de la 4-(2-éthyl-1,3-dioxolan-2-yl)pentan-2-one (composé 4) 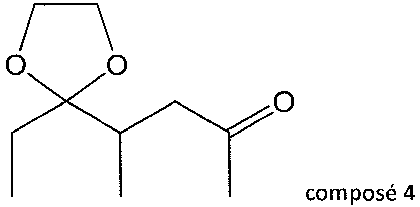 par réaction d'oxydation de la 2-éthyl-2-(pent-4-èn-2-yl)-1,3-dioxolane (composé 3) 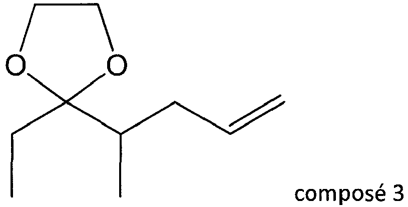 en présence d'un catalyseur choisi dans le groupe comprenant les complexes organométalliques de métaux de transition.

15. Composition de sordidine **caractérisée en ce que** qu'elle comprend les 4 diastéréoisomères a, b, c et d dont les proportions respectives mesurées par chromatographie en phase gazeuse sont comprises dans les intervalles : 31-36% ; 16-20% ;16-20% ; 28-32%

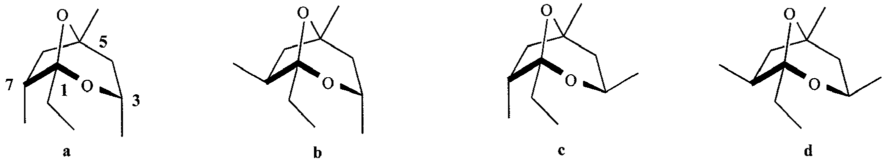

## Patentansprüche

1. Verfahren zur Herstellung von Sordidin, umfassend einen Schritt der Herstellung von 4-(2-Ethyl-1,3-dioxolan-2-yl)pentan-2-on (Verbindung 4) 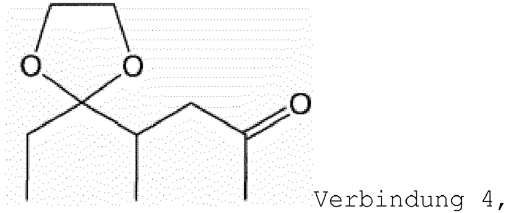 durch Oxidationsreaktion von 2-Ethyl-2-(pent-4-en-2-yl)-1,3-dioxolan (Verbindung 3) 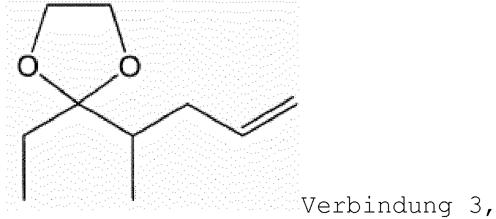 in Gegenwart eines Katalysators, der aus der Gruppe umfassend organometallische Komplexe von Übergangsmetallen ausgewählt ist.

2. Verfahren nach Anspruch 1, **dadurch gekennzeichnet, dass** die Oxidationsreaktion in Gegenwart eines Oxidationsmittels durchgeführt wird, das aus Luft, mit Sauerstoff angereicherter Luft, Sauerstoff O₂ und Hydroperoxiden der allgemeinen Formel R-OOH, wobei R ein Wasserstoffatom, eine lineare oder verzweigte C₁-C₆-Alkylgruppe sein kann, ausgewählt ist.

3. Verfahren nach Anspruch 1 oder 2, **dadurch gekennzeichnet, dass** der Katalysator aus organometallischen Komplexen von Übergangsmetallen auf der Basis von Ni, Rh, Ir, Pd, Co oder Pt ausgewählt ist.

4. Verfahren nach einem der vorhergehenden Ansprüche, **dadurch gekennzeichnet, dass** der Katalysator aus Palladium(II)-Verbindungen, aus Nickel(II)-Verbindungen, aus Kobalt-Verbindungen oder auch aus Rhodium(III)-Verbindungen ausgewählt ist.

5. Verfahren nach einem der vorhergehenden Ansprüche, **dadurch gekennzeichnet, dass** der Katalysator durch einen Liganden komplexiert ist, der aus Aminen, Phosphinen und Phthalocyaninen ausgewählt ist.

6. Verfahren nach einem der vorhergehenden Ansprüche, **dadurch gekennzeichnet, dass** ein Regenerator des Katalysators zugegeben wird.

7. Verfahren nach Anspruch 6, **dadurch gekennzeichnet, dass** der Katalysatorregenerator aus Regeneratoren auf der Basis von Kupfer oder Eisen ausgewählt ist.

8. Verfahren nach einem der Ansprüche 1 bis 4, **dadurch gekennzeichnet, dass** der Katalysator Pd(acetat)₂ ist.

9. Verfahren nach einem der vorhergehenden Ansprüche, **dadurch gekennzeichnet, dass** die Oxidationsreaktion der Verbindung 3 zur Verbindung 4 in Gegenwart von mindestens einem Lösungsmittel vorgenommen wird, das aus Wasser, alkoholischen Lösungsmitteln, sauren Lösungsmitteln, Lösungsmitteln vom Ketontyp, Ether-Lösungsmitteln, Stickstoff-Lösungsmitteln sowie Lösungsmitteln von Flüssigpolymertypen ausgewählt ist.

10. Verfahren nach einem der vorhergehenden Ansprüche, **dadurch gekennzeichnet, dass** die Oxidationsreaktion der Verbindung 3 zur Verbindung 4 in Gegenwart von mindestens einem Lösungsmittel vorgenommen wird, das aus Wasser, DMF, DMA, Aceton, Heptan, tert.-BuOH oder deren Gemisch ausgewählt ist.

11. Verfahren nach einem der vorhergehenden Ansprüche, **dadurch gekennzeichnet, dass** die Oxidationsreaktion unter einem Druck vorgenommen wird, der zwischen atmosphärischem Druck und 30 bar liegt.

12. Verfahren nach einem der vorhergehenden Ansprüche, **dadurch gekennzeichnet, dass** die Oxidationsreaktion bei einer Temperatur vorgenommen wird, die zwischen 25 °C und 100 °C liegt.

13. Verfahren zur Synthese von Sordidin, **dadurch gekennzeichnet, dass** es ein Verfahren nach einem der vorhergehenden Ansprüche, gefolgt von der Umwandlung von 4-(2-Ethyl-1,3-dioxolan-2-yl)pentan-2-on (Verbindung 4) 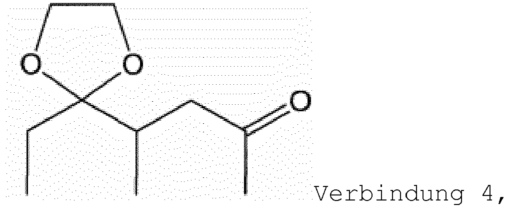 in Verbindung 5 durch Magnesium-Kopplung mit Magnesiumallylhalogenid, dann der Umwandlung der Verbindung 5 in Verbindung 6 durch Oxidation, dann der Umwandlung der Verbindung 6 in Verbindung 7 durch Epoxidöffnung und der Umsetzung der Verbindung 7 in Sordidin durch Ringschluss umfasst.

14. Verfahren zur Herstellung von 4-(2-Ethyl-1,3-dioxolan-2-yl)pentan-2-on (Verbindung 4) 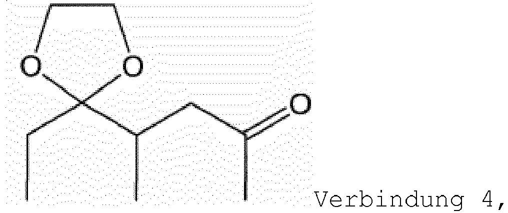 durch Oxidationsreaktion von 2-Ethyl-2-(pent-4-en-2-yl)-1,3-dioxolan (Verbindung 3) 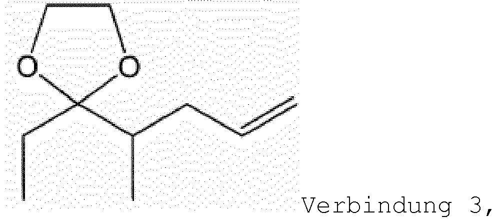 in Gegenwart eines Katalysators, der aus der Gruppe umfassend organometallische Komplexe von Übergangsmetallen ausgewählt ist.

15. Sordidin-Zusammensetzung, **dadurch gekennzeichnet, dass** sie die 4 Diastereoisomere a, b, c und d umfasst, wobei deren jeweilige Anteile, die durch Gasphasenchromatographie gemessen werden, von den folgenden Spannen umfasst werden: 31-36 %; 16-20 %; 16-20 %; 28-32 %

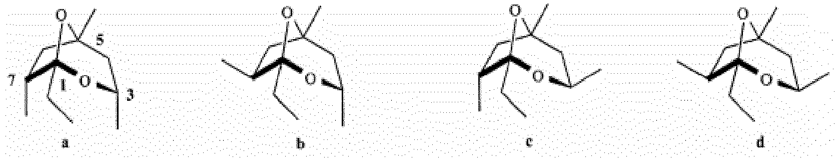

## Claims

1. Process for preparing sordidin comprising a step for preparing 4-(2-ethyl-1,3-dioxolan-2-yl)pentan-2-one (compound 4) 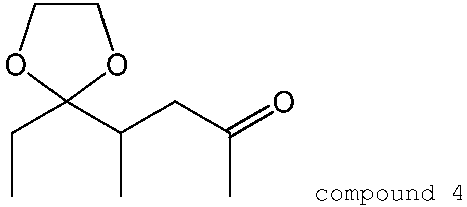 by means of a reaction in which 2-ethyl-2-(pent-4-en-2-yl)-1,3-dioxolane (compound 3) 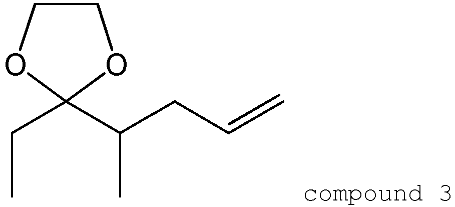 is oxidised in the presence of a catalyst chosen from the group comprising organometallic complexes of transition metals.

2. Process according to claim 1, **characterised in that** the oxidation reaction is carried out in the presence of an oxidising agent chosen from air, oxygen-enriched air, oxygen O₂ and hydroperoxides with the general formula R-OOH wherein R can be a hydrogen atom or a linear or branched C₁-C₆ alkyl group.

3. Process according to claim 1 or 2, **characterised in that** the catalyst is chosen from organometallic complexes of transition metals with an Ni, Rh, Ir, Pd, Co or Pt base.

4. Process according to one of the preceding claims, **characterised in that** the catalyst is chosen from Palladium (II) compounds, from Nickel (II) compounds, from Cobalt compounds or from Rhodium (III) compounds.

5. Process according to one of the preceding claims, **characterised in that** the catalyst is complexed by a ligand chosen from amines, phosphines and phtalocyanines.

6. Process according to one of the preceding claims, **characterised in that** a catalyst regenerator is added.

7. Process according to claim 6, **characterised in that** the catalyst regenerator is chosen from copper or iron-based regenerators.

8. Process according to one of claims 1 to 4, **characterised in that** the catalyst is Pd(Acetate)₂.

9. Process according to one of the preceding claims, **characterised in that** the oxidation reaction of compound 3 into compound 4 is carried out in the presence of at least one solvent chosen from water, alcoholic solvents, acidic solvents, solvents of the ketone type, ether solvents, nitrogen solvents, as well as solvents of the liquid polymer type.

10. Process according to one of the preceding claims, **characterised in that** the oxidation reaction of compound 3 into compound 4 is carried out in the presence of at least one solvent chosen from water, DMF, DMA, acetone, heptane, tertBuOH or mixtures thereof.

11. Process according to one of the preceding claims, **characterised in that** the oxidation reaction is carried out under a pressure between atmospheric pressure and 30 bar.

12. Process according to one of the preceding claims, **characterised in that** the oxidation reaction is carried out at a temperature between 25°C and 100°C.

13. Process for synthesising sordidin **characterised in that** it comprises a process according to one of the preceding claims, followed by the transforming of the 4-(2-ethyl-1,3-dioxolan-2-yl)pentan-2-one (compound 4) 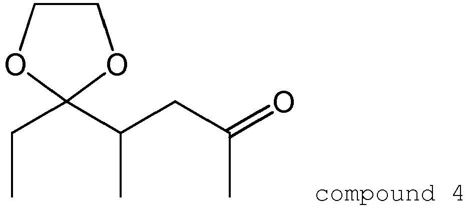 into compound 5 by magnesium coupling with allyl magnesium halide, then the transforming of compound 5 into compound 6 by oxidation, then the transforming of compound 6 into compound 7 by an opening of the epoxide, and converting of the compound 7 into sordidin by cyclisation.

14. Process for preparing 4-(2-ethyl-1,3-dioxolan-2-yl)pentan-2-one (compound 4) 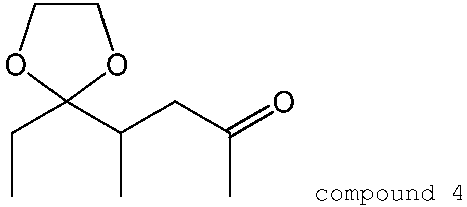 by means of a reaction in which 2-ethyl-2-(pent-4-en-2-yl)-1,3-dioxolane (compound 3) 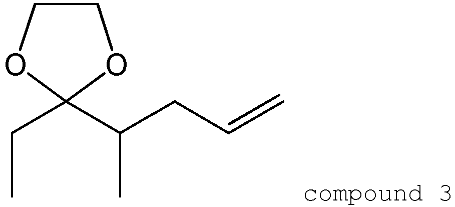 is oxidised in the presence of a catalyst chosen from the group comprising organometallic complexes of transition metals.

15. Composition of sordidin **characterised in that** it comprises the 4 diastereoisomers a, b, c and d of which the respective proportions measured by gas phase chromatography are comprised in the intervals: 31-36%; 16-20%; 16-20%; 28-32%
